# EUROPEAN PATENT APPLICATION

(11) **EP 4 066 767 A1**
(43) Date of publication of application: **05.10.2022**
(21) Application number: 22166094.7
(22) Date of filing: 31.03.2022
(51) Int. Cl.: A61B 18/14, A61B 18/00, A61N 1/04, A61N 1/06

(54) **BODY CONTOURING DEVICE USING RF ENERGY, CONTROL METHOD THEREOF AND BODY CONTOURING METHOD USING THE SAME**

(30) Priority: 31.03.2021 US 202163168821 P
(71) Applicant: Lutronic Corporation, Goyang-si, Gyeonggi-do 10534 (KR)
(72) Inventor: BARTHOLOMEUSZ, James, Beverly Hills, CA, 90210 (US); KO, Kwang Chon, 10893 Paju-si, Gyeonggi-do (KR)
(74) Representative: Michalski Hüttermann & Partner Patentanwälte mbB

(57) **Abstract**

Disclosed are a body contouring device using RF energy, a control method thereof, and a body contouring method using the same, in which a surface of tissue overheated due to the edge effect is selectively cooled while the tissue is heated with the RF energy transferred thereto, thereby having a uniform treatment effect on a treatment area, reducing pain, and preventing the tissue from being damaged.

## Description

### BACKGROUND

### Field

The disclosure relates to a body contouring device using radio frequency (RF) energy, a control method thereof, and a body contouring method using the same.

### Description of the Related Art

In the fields of medicine, radio frequency (RF) energy has been widely used for the purpose of removing or excising tissue in such a way that the tissue is heated by the RF energy locally transferred thereto.

However, the RF energy has recently been developed and used in a tissue remodeling method to treat aging skin tissue (wrinkle, weak skin elasticity, etc.). Besides, the RF energy has also been applied to wide-ranging subcutaneous fat to kill fat cells. Like this, the application range of the RF energy is expanding in the medical fields.

However, the related art has a problem in that the tissue is unintentionally damaged as electrodes are overheated by the edge effect when the RF energy is applied through skin.

### SUMMARY

The disclosure is to provide a body contouring device using radio frequency (RF) energy, a method thereof, and a body contouring method using the same, in which tissue is prevented from being damaged by an edge effect, and pain is reduced when body contouring is performed using the RF energy.

The disclosure is to provide a body contouring device using radio frequency (RF) energy, which includes a plurality of divided electrodes to minimize the edge effect while transferring the RF energy, and a cooler configured to intensively cool the electrode where the edge effect occurs.

Further, the disclosure is to provide a method of controlling a body contouring device using RF energy, which includes transferring the RF energy to each of a plurality of divided electrodes to minimize the edge effect during the transfer of the RF energy, adjusting a cooling parameter according to the frequencies of the transferred RF energy, and controlling the cooler to intensively cool the electrode where the edge effect occurs.

In addition, the disclosure is to provide a body contouring method using RF energy, which includes intensively cooling tissue partially heated by the edge effect to prevent the tissue from being damaged by the edge effect while body contouring is performed using the RF energy.

According to the disclosure, a body contouring device using radio frequency (RF) energy, a method thereof, and a body contouring method using the same have effects on preventing tissue from being damaged by an edge effect, reducing a patient's pain, and maximizing a treatment effect when the RF energy is transferred through electrodes.

### BRIEF DESCRIPTION OF THE DRAWINGS

The above and/or other aspects will become apparent and more readily appreciated from the following description of embodiments, taken in conjunction with the accompanying drawings, in which:
FIG. 1 is a perspective view showing an electrode pad according to a first embodiment of the present invention;
FIG. 2 is an exploded perspective view of an electrode pad according to the first embodiment of the present invention;
FIG. 3 is a bottom view of the electrode pad of FIG. 1;
FIG. 4 is an enlarged cross-sectional view taken along line I-I' of FIG. 3;
FIG. 5 is a conceptual diagram showing a temperature distribution within a tissue when an electrode pad according to a first embodiment of the present invention is used;
FIG. 6 is a partial cross-sectional view of an electrode pad according to a second embodiment of the present invention;
FIG. 7 is a conceptual diagram showing a state of use of an electrode pad according to a second embodiment of the present invention;
FIG. 8 is a conceptual diagram reconstructed from an electrical point of view when an electrode pad according to the second embodiment of the present invention is used;
FIG. 9 illustrates thermal distribution of an electrode pad when RF energy having different frequencies is transferred to tissue;
FIG. 10 is a perspective view of a body contouring device using RF energy according to a third embodiment of the disclosure;
FIG. 11 is a block diagram of the body contouring device using the RF energy according to the third embodiment of the disclosure;
FIG. 12 is an exploded perspective view of an applicator according to the third embodiment;
FIG. 13 illustrates an operation state of a cooler according to the third embodiment;
FIG. 14 illustrates temperature of an electrode pad according to operations of a cooler in the third embodiment;
FIG. 15 is a flowchart of a method of controlling a body contouring device using RF energy according to a fourth embodiment of the disclosure;
FIG. 16 is a flowchart of a method of controlling a body contouring device using RF energy according to a fifth embodiment of the disclosure;
FIG. 17 is a flowchart of a body contouring method using RF energy according to a sixth embodiment of the disclosure; and
FIG. 18 is a detailed flowchart of the body contouring method using the RF energy according to the sixth embodiment of the disclosure.

### DETAILED DESCRIPTION OF EMBODIMENTS

Hereinafter, body contouring device using rf energy, control method thereof and body contouring method using the same according to an embodiment of the present invention will be described in detail with reference to the accompanying drawings. In addition, in the description of the following embodiments, the names of each component may be referred to by other names in the art. However, if a modification is employed, when there are functional similarity and sameness, components thereof may be considered to be the same. In addition, reference numerals added to each component are used for convenience of description. However, the content illustrated on the drawings in which these reference numerals are indicated does not limit each component to the range within the drawings. Likewise, even if an embodiment in which some components in the drawings are partially modified is employed, if there is functional similarity and sameness, the components may be considered to be the same. In addition, in view of the level of a general technician in the relevant technical field, if a component is recognized as a component that should be naturally included, a description thereof will be omitted.

Meanwhile, hereinafter, body contouring refers to a treatment based on transferring energy to tissue to make destruction in units of cells. For instance, the body contouring may be a treatment in which RF energy is transferred for heating to kill fat cells during the treatment, and subcutaneous fat is prevented from being formed in a treated area by the fat cells for a long period of time, thereby changing a body shape.

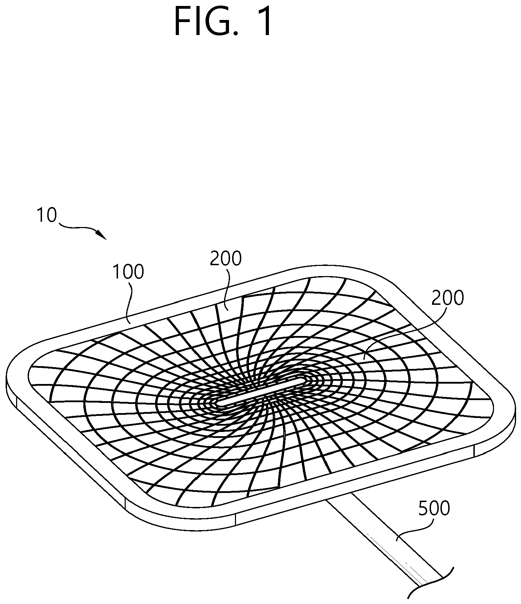

Hereinafter, a configuration of body contouring electrode pad according to a first embodiment of the present invention will be described in detail with reference to FIGS. 1 to 4.

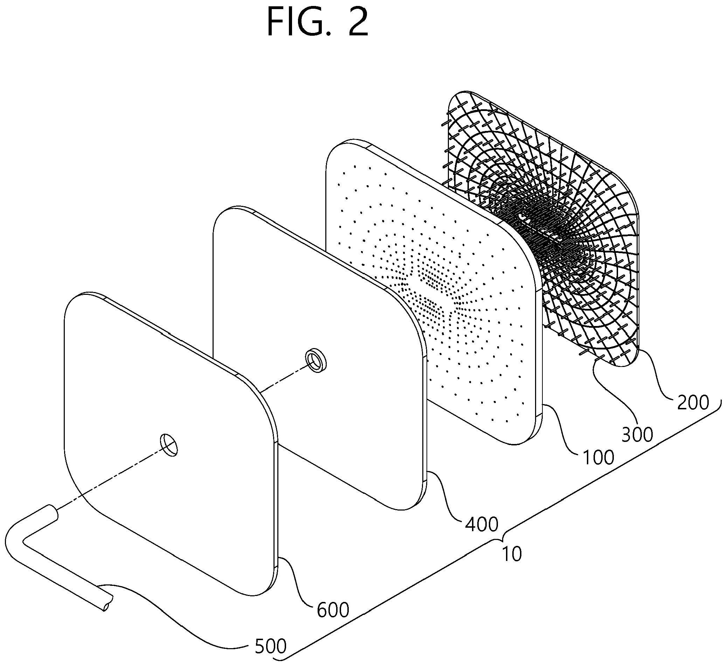

FIG. 1 is a perspective view showing an electrode pad according to the first embodiment of the present invention, and FIG. 2 is an exploded perspective view of the electrode pad according to the first embodiment of the present invention.

Referring to FIG. 1, the electrode pad 10 according to the first embodiment of the present invention may be configured to be attached to a skin surface and may receive RF energy from the outside in a state of being attached to the skin and transfer the received RF energy to the skin.

An electrode pad 10 according to the first embodiment of the present invention may include a base 100, an electrode 200, a first connection portion 300, a second connection portion 400, a shielding layer 600, and a connector 500.

The base 100 is a base on which the electrode 200, the first connection portion 300, and the second connection portion 400 are disposed. The base 100 may be entirely formed in a flat plate shape. The base 100 is configured in a flat plate shape having a large upper or lower surface in FIG. 1, and a plurality of electrodes 200 may be provided as a planar arrangement on the upper or lower surface. The base 100 may be formed of an insulating material so that current may not flow through the base 100 between the electrodes when the electrodes 200 to be described later are divided to be arranged and RF energy is applied from the outside.

Hereinafter, it is assumed that a plurality of electrodes 200 is provided on the lower surface 101 of the base.

The electrode 200 may configured to be airtightly in contact with a skin when transferring RF energy applied from the outside to the skin. Each electrode 200 having flat plate shape is configured such that an upper surface thereof is in contact with the base 100 and a lower surface thereof is in contact with the skin. Therefore, when the base 100 is in close contact with the skin, the plurality of electrodes 200 may be in contact with the skin at a plurality of points to transfer RF energy.

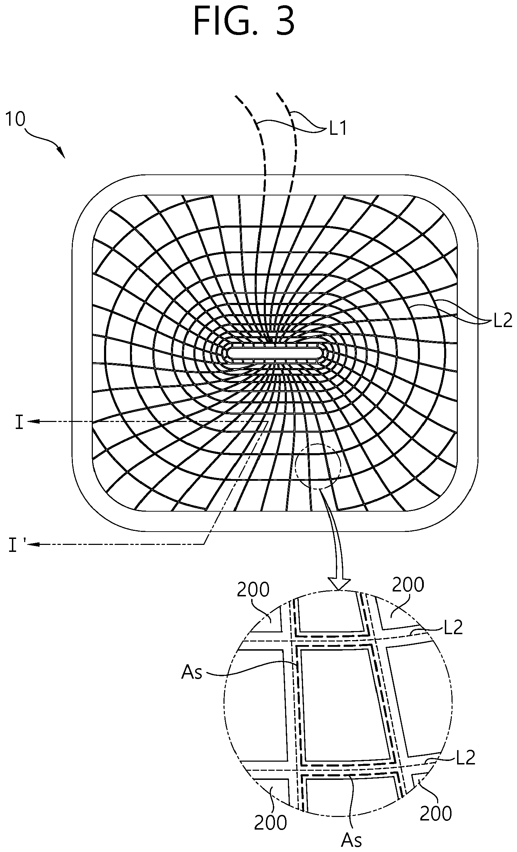

The electrode 200 is provided in plurality and the plurality of electrodes may be arranged in planar manner on the lower surface 101 of the base. The electrodes 200 may be disposed to be spaced apart from each other by a predetermined distance on the lower surface 101 of the base. Meanwhile, the planar arrangement and the shape of each electrode 200 will be described in detail later with reference to FIG. 3.

The first connection portion 300 is configured to be electrically connected to the plurality of electrodes 200 provided on the lower surface 101 of the base. The first connection portion 300 may be configured to penetrate the base 100 in a thickness direction, that is, from an upper surface to a lower surface. The first connection portion 300 is configured in the form of a pin and provided in a number corresponding to the number of the plurality of electrodes 200, so that the plurality of first connection portions may be electrically connected to the plurality of electrodes 200, respectively on one side thereof. As an example, the first connection portion 300 may be configured as a single member extending from the upper surface of each of the plurality of electrodes 200 by a predetermined length, and the predetermined length of each of the first connection portions 300 may be greater than a thickness of the base 100. In this case, when the plurality of electrodes 200 are installed on the base 100, an upper end of the first connection portion 300 may be exposed on an upper surface 102 of the base. However, in the present embodiment, an example in which the first connection portion 300 is formed of a pin has been described, but the shape of the first connection portion 300 may be modified and applied as various components that may be electrically connected to each electrode 200.

The second connection portion 400 is configured to transfer RF energy applied from the outside to the plurality of first connection portions 300. The second connection portion 400 may be provided on the upper surface 102 of the base and may be configured to be electrically connected to upper ends of the plurality of first connection portions 300 described above at a plurality of points. The second connection portion 400 may be configured such that one side thereof is electrically connected to the connector 500 to be described later to receive RF energy from the outside. For example, the second connection portion 400 may be formed of a metal pad having a flat plate shape. In this case, a lower surface of the metal pad may be in close contact with the upper surface 102 of the base and may be electrically connected to the plurality of first connection portions 300 at a plurality of points. Meanwhile, an example in which the second connection portion 400 is formed of a metal pad has been described above, but the second connection portion 400 may be applied as various components such as an electrical element, e.g., a metal mesh, a metal wire, or the like which may be electrically connected to an end of the plurality of first connection portions 300 and receive RF energy from the outside.

The shielding layer 600 is configured to cover the second connection portion 400 exposed on the upper surface 102 of the base. The shielding layer 600 may be configured in the form of a film to cover the second connection portion 400 and may be configured to insulate the second connection portion 400 from the outside.

A base 100, a first connecting portion 300, and a second connecting portion 400 may be provided with cooling channels in which a cooling fluid flows. The cooling channels are structured to cool the base 100, the electrode 200, the first connecting portion 300 and the second connecting portion 400 while the cooling fluid introduced from a connector (to be described later) is flowing therein. A fluid channel may be at least partially disposed in an outer boundary portion of the electrode array so that at least a portion of an area where an edge effect occurs can be cooled. There may be a plurality of cooling channels dividing an area corresponding to the electrode array into areas of a certain size, and structured to independently cool the divided areas, respectively.

The connector 500 is configured to receive RF energy from the outside. The connector 500 may be provided on the upper surface 102 of the base, may be provided in a region exposed to the upper side of the shielding layer 600 so that one side thereof may be electrically connected to the second connection portion 400. For example, the connector 500 may be provided at a center portion of the upper side of the shielding layer 600, and one side thereof may be connected to the second connection portion 400 through the shielding layer 600. However, the configuration and installation position of the connector 500 described above are merely an example, and the connector 500 may be modified and applied as various components that may electrically connect the outside and the second connection portion 400.

Meanwhile, although not shown, the electrode pad 10 may be connected with an RF energy generating device capable of generating RF energy such as an RF generator, an RF modulator, and an impedance matching circuit, so as to be used.

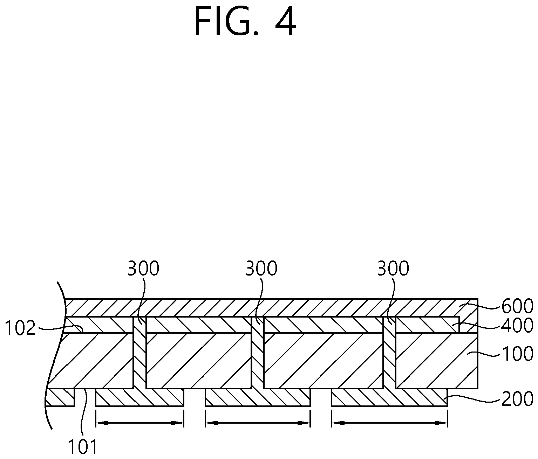

Hereinafter, the electrode 200 of the present embodiment will be described in detail with reference to FIGS. 3 and 4.

FIG. 3 is a bottom view of the electrode pad 10 of FIG. 1. As shown, in the present embodiment, a plurality of electrodes 200 may be provided in sections 3000 divided on a lower surface of the base 100, respectively.

A lower surface 101 of the base may be divided into sections 3000 by a first virtual line 1000 and a second virtual line 2000. The first virtual line 1000 may be formed radially from a center portion of the base 100 toward an outer edge portion. The first virtual line 1000 may is provided in plurality, and the plurality of first virtual lines 1000 may be arranged to be spaced apart from each other at a predetermined angle in a rotation direction based on the center portion of the base 100. At least a portion of each of the first lines 1000 may be formed of a curved line. As an example, the first line 1000 may be configured to have curve in a sinusoidal wave-shape. In this case, each of the first lines 1000 may be formed to have a length shorter than one wavelength of the sinusoidal wave. That is, as shown in FIG. 3, one first line 1000 may have a length in which the sinusoidal wave waveform is not completed from the center portion of the base 100 to an outer rim of the base 100, that is, a length shorter than the wavelength.

The second virtual line 2000 may be formed along an annular path surrounding the center portion on the lower surface 101 of the base. As an example, the second virtual line 2000 may have a stadium shape as a whole, and may be configured to form a closed path. A plurality of second virtual lines 2000 are defined and formed concentrically with each other, and a space between the second virtual lines 2000 increases in a direction toward the outer edge of the base 100.

The electrodes tend to gradually decrease in size in a direction toward the center on the lower surface of the base. Here, a minimum size of the electrode may be limited. Thus, at least a portion of the center portion of the lower surface of the base may not have an electrode. However, this is only an example, and at least a portion of the center portion on the lower surface of the base may be modified and applied as a configuration in which electrodes having a uniform size are disposed.

The lower surface 101 of the base may be divided into a plurality of virtual sections 3000 by a plurality of first and second lines 1000 and 2000. A plurality of electrodes 200 may be arranged in the divided sections 3000 of the lower surface 101 of the base, excluding the first virtual line 1000 and the second virtual line 2000. Here, since the first line 1000 is configured in the shape of a sinusoidal wave, at least a portion of a boundary edge of each of a plurality of regions may be configured as a curved line.

Referring to a partially enlarged region of FIG. 3, a plurality of electrodes 200 may be arranged in sections 3000, respectively. A planar shape of the plurality of electrodes 200 may be determined to correspond to a shape of the section 3000 determined by the first line 1000 and the second line 2000. Here, since the first lines 1000 are arranged radially as a whole, a space between each of the first lines 1000 increases in a direction away from the center portion. Accordingly, the size of the plurality of electrodes 200 varies depending on the position at which the electrodes are arranged. As an example, as shown in FIG. 3, the size of the electrode 200 gradually increases in a direction toward the outer edge on the lower surface 101 of the base.

FIG. 4 is an enlarged cross-sectional view taken along line I-I' of FIG. 3. As illustrated, a plurality of electrodes 200 different from each other may be provided on the lower surface of the base 100. One side of the first connection portion 300 may be connected to each electrode 200, the first connection portion 300 penetrate the base 100, and the other side thereof may be connected to the second connection portion 400. The shielding layer 600 may be provided on the second connection portion 400.

Meanwhile, the electrode pad 10 according to the present invention described above may be entirely formed of a rigid material. When the electrode pad 10 is formed of a rigid material, the electrode pad 10 may be easily attached to a part of the skin with high flatness, such as the pectoralis major muscle and the thigh muscle.

Meanwhile, the electrode pad 10 according to the present invention may be entirely formed of a flexible material. When the electrode pad 10 is formed of a flexible material, it is possible to increase adhesion when the electrode pad 10 is attached to the skin in order to stimulate a part of the skin with low flatness, for example, muscles in arms and calves.

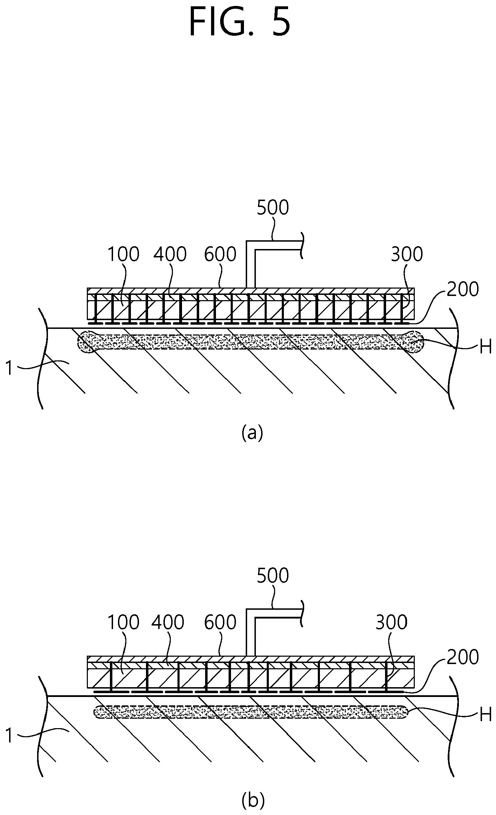

FIG. 5 is a conceptual diagram showing a temperature distribution in a tissue 1 when the electrode pad 10 according to the first embodiment of the present invention is used.

Referring to FIG. 5A, when RF energy is applied using the electrode pad 10, an edge effect in which current is concentrated on the edge portion occurs. When a muscle is stimulated by applying current, a phenomenon in which a heating portion H is unnecessarily concentrated due to a resistance component of the tissue 1 itself in a movement path of the current occurs. When the current is concentrated to flow in the tissue 1 by the edge effect, a temperature rises intensively in a part, causing a problem that damage and pain of the tissue 1 are increased.

Therefore, it is desirable to minimize the concentration of current and apply current uniformly to each part in a state where the electrode pad 10 is attached to the skin. Referring to FIG. 5B, according to the present invention, a size of the plurality of electrodes 200 gradually increases in a direction from the center portion toward the outer edge. In addition, since each electrode 200 is formed along a sinusoidal wave, which is a shape of the first line 1000, current may be uniformly applied as a whole. Eventually, the electrode pad 10 according to the present invention has a difference in size of each electrode 200 and a difference in shape of each electrode 200, so that uniform current may be applied throughout when the plurality of electrodes 200 are simultaneously used in a state of being arranged. Therefore, it is possible to evenly distribute the heating portion H by RF energy in the tissue 1.

Meanwhile, the electrode pad 10 according to the present invention stimulates the muscle upon receiving RF energy from the outside, and here, RF energy may be transferred in a monopolar or bipolar manner. In the case of transferring RF energy in the monopolar manner, a separate ground electrode 200 may be used together. Meanwhile, when RF energy is configured to be transferred in the bipolar manner, the electrode pads 10 may be configured as a pair and may be simultaneously attached to the skin and used.

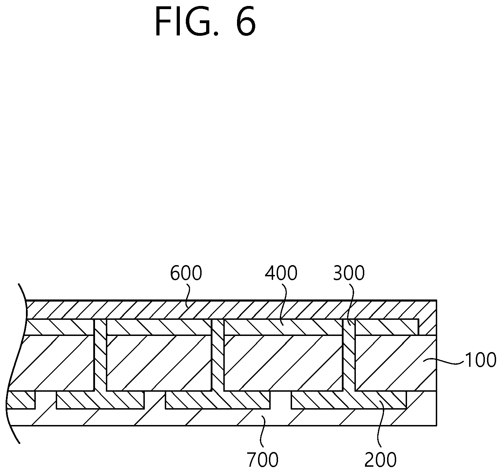

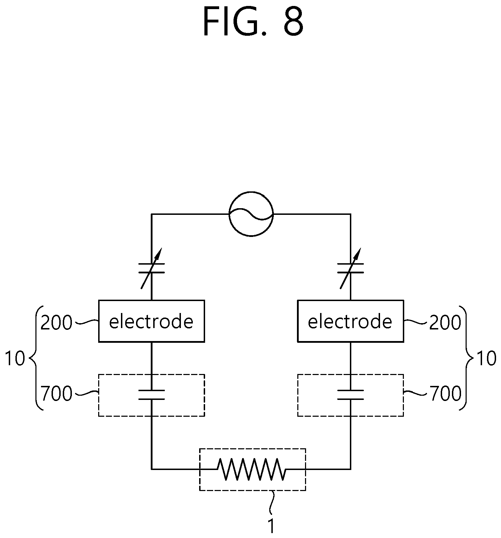

Hereinafter, an electrode pad 10 according to a second embodiment of the present invention will be described in detail with reference to FIGS. 6 and 8.

This embodiment may also be configured to include the same components as those of the embodiment described above, and descriptions of the same components will be omitted to avoid redundancy and different components will be described.

FIG. 6 is a partial cross-sectional view of the electrode pad 10 according to the second embodiment of the present invention. The second embodiment of the present invention may include a dielectric layer 700 covering a plurality of electrodes 200 provided on a lower surface 101 of a base.

The dielectric layer 700 is formed in a flat plate shape and may be configured to cover a plurality of electrodes 200 at the same time. The dielectric layer 700 may be formed of a material having a dielectric constant in a predetermined range. The dielectric layer 700 may be attached to each of the electrodes 200 such that an upper surface thereof covers the electrodes 200 and a lower surface thereof is attached to the skin. The dielectric layer 700 may be formed of a rigid or flexible material. As an example, the dielectric layer 700 may be formed of ceramic or polytetrafluoroethylene (PTFE).

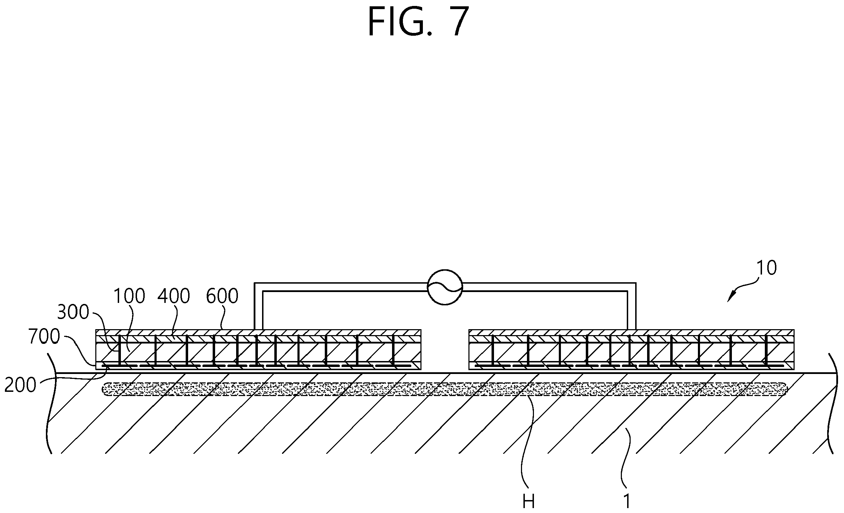

FIG. 7 is a conceptual diagram showing a use state of the electrode pad 10 according to the second embodiment of the present invention. In this embodiment, the concept of transferring RF energy in a bipolar manner is disclosed, and in this case, RF energy is transferred to stimulate the muscle in a state where a pair of electrode pads 10 is attached to the skin. The muscle contracts as RF energy is transferred to the tissue 1 between the pair of EMS electrodes 200. Here, the RF energy may have a frequency of 2 to 10 NHrz, and it is possible to maximize electrical stimulation of the muscle, while distributing heating points in the tissue 1.

Here, when the lower surface of the electrode pad 10 is coated with the dielectric layer 700, capacitive coupling may be formed between each electrode 200 and the skin. The dielectric layer 700 functions as a capacitor between the electrode 200 and the skin when RF energy is applied to the skin using the electrode pad 10. As a result, since capacitive coupling is formed with the tissue 1 at the end of each electrode 200, an influence of parasitic capacitance may be minimized. In addition, it is possible to minimize an edge current in which an unintended overcurrent occurs in the electrode 200 disposed at the edge portion of the arrangement of the plurality of electrodes 200.

FIG. 8 is a conceptual diagram reconstructed from an electrical point of view when the electrode pad 10 according to the second embodiment of the present invention is used.

Referring to FIG. 8, it is electrically connected so that RF energy may be transferred to the electrode pad 10 from an external RF energy generating device. The electrode pad 10 transfers RF energy through the plurality of electrodes 200, and here, each dielectric layer 700 functions as a capacitor in the tissue 1 expressed as a resistor. When RF energy is applied, impedance matching is performed using a variable capacitor or the like provided in the RF energy generating device, and here, accuracy of impedance matching may be improved by a capacitance component of the dielectric layer 700. After the impedance matching is completed, the RF energy generating device transfers RF energy to the electrode pad 10, and RF energy is finally transferred to the tissue 1 to stimulate muscle.

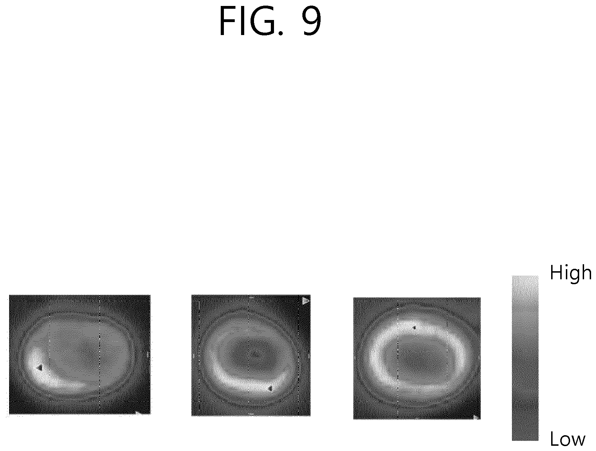

FIG. 9 illustrates thermal distribution of an electrode pad when RF energy having different frequencies is transferred to tissue.

FIG. 9 shows a thermal image displayed when the RF energy having different frequencies is transferred to the tissue through the electrode pad according to the first to third embodiments of the disclosure.

Referring to FIG. 9, a heating area, penetration depth, and temperature distribution may be varied depending on the frequencies of the RF energy. When temperature is measured while changing the frequency of the RF energy into 2, 7 and 13 MHz but maintaining other conditions than the frequency, different temperature change patterns are shown. It is known that the higher the frequency of the RF energy, the shallower the penetration depth when transferred through a surface, and, on the other hand, the lower the frequency, the deeper the penetration depth.

Meanwhile, a sharp rise in temperature of tissue may shorten treatment time but increase pain, and difference in rate of temperature rise according to areas of tissue may cause the tissue to be overheated and unintentionally damaged. Therefore, it is necessary to transfer the RF energy by adjusting the parameters of the RF energy in order to maximize a treatment effect and prevent the tissue from being unintentionally damaged. For example, the parameters of the RF energy, such as frequencies, power, applying periods of time, etc. are adjustable.

As an example of the temperature change patterns based on the parameter adjustment, first, pre-heating may be performed for a predetermined period of time by selecting a frequency of 2 MHz. Then, the frequency may be adjusted into 7 MHz to perform high heating, or 13 MHz to perform fat layer heating. Further, the frequency may be adjusted in order of 2-7-13 MHz to heat tissue.

Referring back to FIG. 9, it is shown that the edge effect increases as the frequency become higher, in other words, a temperature gradient is generated as energy is concentrated in an outer boundary portion of an electrode array. When the tissue is partially overheated, pain may increase and the tissue may be unintentionally damaged.

Accordingly, an electrode in which the edge effect is concentrated may be cooled to prevent tissue from being partially damaged by the edge effect.

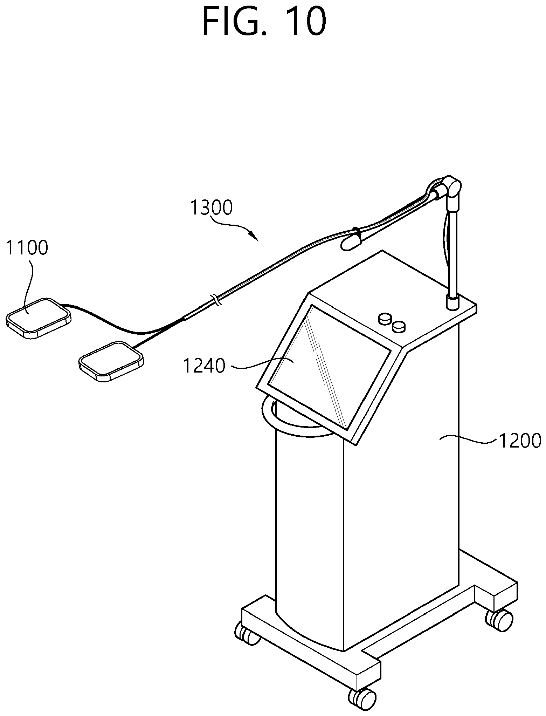

Below, a body contouring device using RF energy according to a third embodiment will be described with reference to FIGS. 10 to 14.

FIG. 10 is a perspective view of the body contouring device using the RF energy according to the third embodiment of the disclosure.

The body contouring device using the RF energy according to the third embodiment of the disclosure may include at least one body applicator 1100, and a main body 1200. Each body applicator 1100 may include the electrode pad according to the first embodiment. Further, each body applicator 1100 may internally include a cooling block to intensively cool a portion where the edge effect occurs in the electrode pad.

One side of each body applicator 1100 is connected to a cable 1300 and thus electrically and fluidically communicated to the main body 1200.

The main body 1200 may internally include an RF generator, a cooler, and a controller. Further, the main body 1200 may include a display 1240 to monitor a current operation situation, and a condition of a person to be treated.

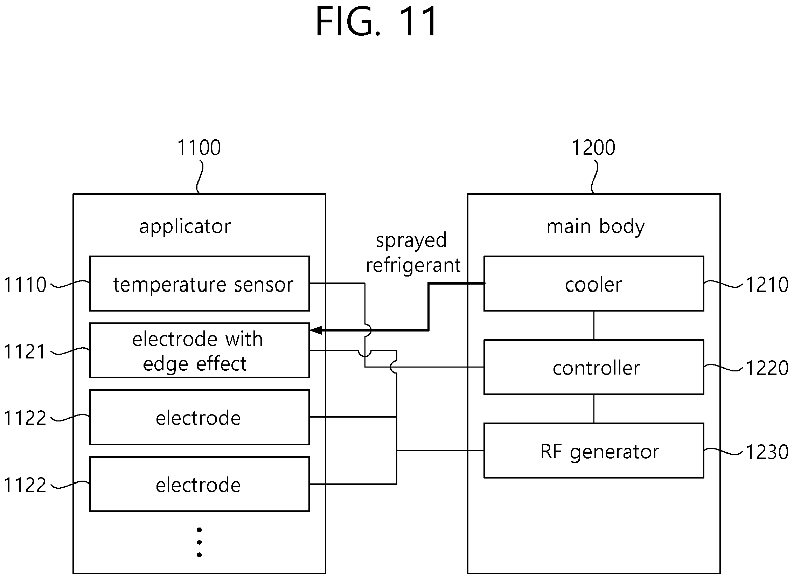

FIG. 11 is a block diagram of the body contouring device using the RF energy according to the third embodiment of the disclosure.

Referring to FIG. 11, of the body contouring device using the RF energy according to the third embodiment of the disclosure may operate interlocking with the applicator 1100.

There may be a plurality of applicators 1100 configured to heat tissue with the RF energy received from the main body 1200.

The applicator 1100 may include a plurality of electrodes 1122 and a temperature sensor 1110. The plurality of electrode 1122 may be the electrodes 1122 provided in the electrode pad described in the first and second embodiments. The temperature sensor 1110 is configured to measure temperature at at least one point of the applicator 1100. For example, a plurality of temperature sensors 1110 may be placed at a point adjacent to an electrode 1121 where the edge effect occurs and at a point adjacent to the electrode 1122 where the edge effect does not occur. The plurality of temperature sensors 1110 may transmit a measured temperature value for identifying whether there is a partial difference in temperature due to the edge effect. However, such positions of the temperature sensors 1110 are merely an example, and the temperature sensors 1110 may be positioned to measure or calculate the temperature of the electrode 1121 where the edge effect occurs, and may, for instance, be disposed on a base, a first connecting portion or a second connecting portion.

The main body 1200 may include an RF generator 1230, a cooler 1210, and a controller 1220.

The RF generator 1230 is configured to receive power from the outside and generate the RF energy. The RF energy generated in the RF generator 1230 may be transmitted to each of the plurality of electrodes 1121 and 1122.

The cooler 1210 is configured to cool the electrode 1121, in which the edge effect is concentrated, among the plurality of electrodes 1122 provided in the applicator 1100. The cooler 1210 includes a refrigerant storage and a valve, and may include a channel through which a refrigerant flows. The cooler 1210 sprays the refrigerant toward the electrode 1121 where the edge effect occurs. In this case, the refrigerant evaporates absorbing heat from the surroundings so that the electrode 1121 can be cooled. Here, the electrode 1121 where the edge effect occurs may be cooled in such a way that the refrigerant is sprayed toward the base provided with the electrode 1122. That is, the electrode 1121 is ultimately cooled as the base is cooled even though the refrigerant is not directly sprayed to the electrode 1121.

The controller 1220 is configured to control the RF generator 1230 to generate the RF energy according to a preset treatment sequence, and control the cooler 1210 to operate based on the measured temperature value.

The controller 1220 controls the cooler 1210 by adjusting the parameter of the cooler 1210 based on the measured temperature value. Further, the controller 1220 may adjust the cooling parameter based on the frequency of the RF energy being currently transferred. Here, the cooling parameter may be related to whether to spray the refrigerant and a spraying duration time of the refrigerant.

Meanwhile, although it is not shown, the main body 1200 may display an input, a current treatment sequence, and the like information on the display. Further, the main body 1200 may include an impedance matcher (not shown) for impedance matching with the electrode 1122 of the applicator 1100 and the like element for transferring the RF energy, and a circuit for modulating and transferring the RF energy.

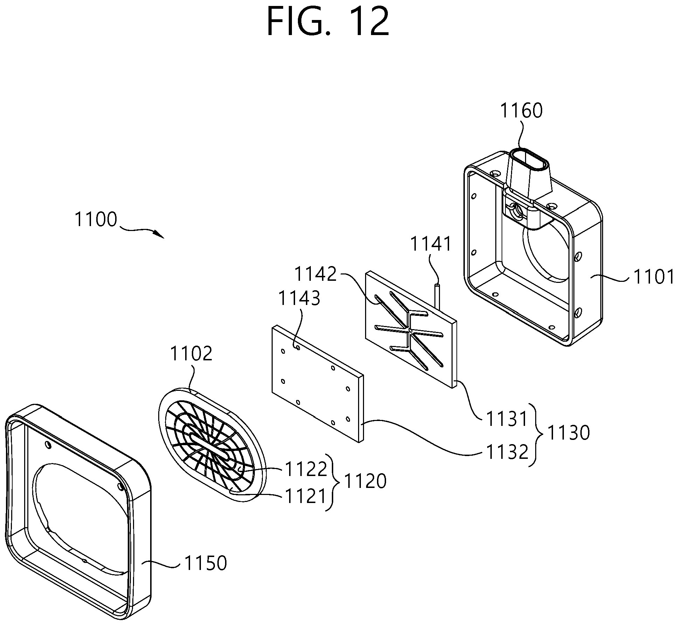

FIG. 12 is an exploded perspective view of the applicator according to the third embodiment.

Referring to FIG. 12, the applicator 1100 according to the third embodiment includes the electrodes 1120 arrayed on one surface thereof, and exposed to be in close contact with skin.

The applicator 1100 may include a housing 1101, a base 1102, the electrodes 1120, a cooling block 1130, and a lower cover 1150.

The housing 1101 is provided to accommodate the base 1102, the electrodes 1120, and the cooling block 1130 therein. The housing 1101 may include a connector 1160 at one side thereof to be electrically and fluidally connected to the main body.

The base 1102 is shaped like a flat plate on which the electrodes 1120 are arrayed. As described in the first or second embodiment, the plurality of electrodes 1120 may be arrayed on the bottom surface of the base 1102. The electrodes 1120 are electrically connected to the inside of the housing 1101 through the connecting portion formed penetrating the base 1102.

The cooling block 1130 is interposed between the housing 1101 and the base 1102, and includes an inlet 1141 formed at one side thereof to connect with the connector so that the refrigerant can be introduced from the outside.

The cooling block 1130 may include a first cooling block 1131 and a second cooling block 1132. The first cooling block 1131 and the second cooling block 1132 may be formed with an internal channel on the sides thereof facing each other. The first cooling block 1131 is formed with a channel at a center portion in a thickness direction, and a groove branched off from the center portion on the surface facing the second cooling block 1132. Each groove may be formed to have a predetermined length toward outer edges on the first cooling block 1131. In this case, the grooves may be formed to have substantially the same length.

The second cooling block 1132 may be formed with a plurality of outlets in a thickness direction. The outlets may be positioned to communicate with the grooves, respectively.

Meanwhile, the cooling block 1130 is structured to make the refrigerant be introduced into the inlet 1141, branched off along the plurality of internal channels 1142, and then sprayed through the outlets 1143 when the cooler of the main body operates. In this case, distances from one inlet 1141 to the outlets 1143 via the internal channels 1142 may be substantially the same.

Meanwhile, the plurality of electrodes 1120 may be different from one another in cooling quantity according to the positions of the outlets 1143.

However, the foregoing structure of the cooling block 1130 is merely an example, and may be variously modified as long as the outlets can spray the refrigerant under substantially the same condition.

The lower cover 1150 is structured to couple with the housing and support internal elements. Although not shown, the lower cover 1150 may include a sucker connecting with a vacuum pump of the main body to provide negative pressure. The sucker may suck in air using a space between the lower cover 1150 and the housing, and secure a fixing force after the applicator 1100 is in close contact with skin.

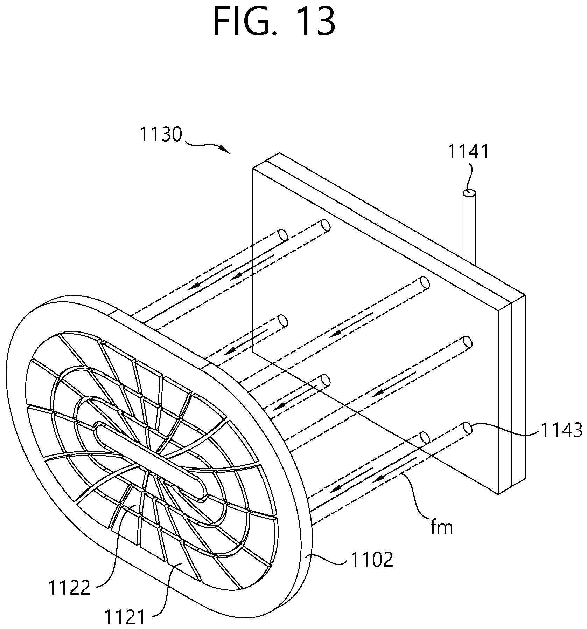

FIG. 13 illustrates an operation state of the cooler according to the third embodiment.

Referring to FIG. 13, the refrigerant introduced from the outside (i.e., the main body) into the inlet 1141 may be branched off in the cooling block 1130 and sprayed toward the electrodes at a plurality of points.

In this case, the outlets 1143 may be formed at the positions corresponding to the plurality of electrodes 1121 where the edge effect occurs, that is, the electrodes 1121 arrayed along the outermost contour in the array of the plurality of electrodes 1120.

Meanwhile, the plurality of outlets 1143 may be spaced apart from one another along the foregoing outermost contour. Although all the plurality of electrodes 1121 arrayed along the outermost contour are not sprayed with the refrigerant fm through the plurality of outlets 1143, the outermost electrodes 1121 where the edge effect occurs are intensively cooled as the refrigerant fm flows.

The refrigerant is sprayed via the outlet 1143 to the electrodes 1121 where the edge effect occurs, so that the electrodes 1121 can be ultimately cooled as the base is substantially cooled.

Further, a spraying duration time of the refrigerant fm may be increased to have a sufficient cooling effect even when the electrodes 122 in the center portion where the edge effect does not occur are overheated as the RF energy is transferred,

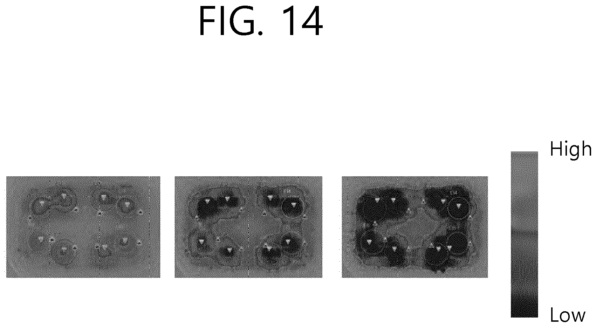

FIG. 14 illustrates the temperature of the electrode pad according to operations of the cooler in the third embodiment.

Referring to FIG. 14, the refrigerant sprayed from the cooling block intensively cools the outermost electrodes where the edge effect occurs. In this case, the temperature may be varied depending on the spraying duration time of the refrigerant from the cooler. In this case, the operations of the cooler are performed by adjusting the parameters based on a value measured by the temperature sensor.

Eventually, in the case where the edge effect occurs even though the electrodes divided to minimize the edge effect are used when the RF energy is transferred to the tissue through the electrodes, the cooler is used to partially cool the electrodes. Accordingly, it is possible to prevent the tissue from being damaged, while having an effect on uniformly treating the tissue.

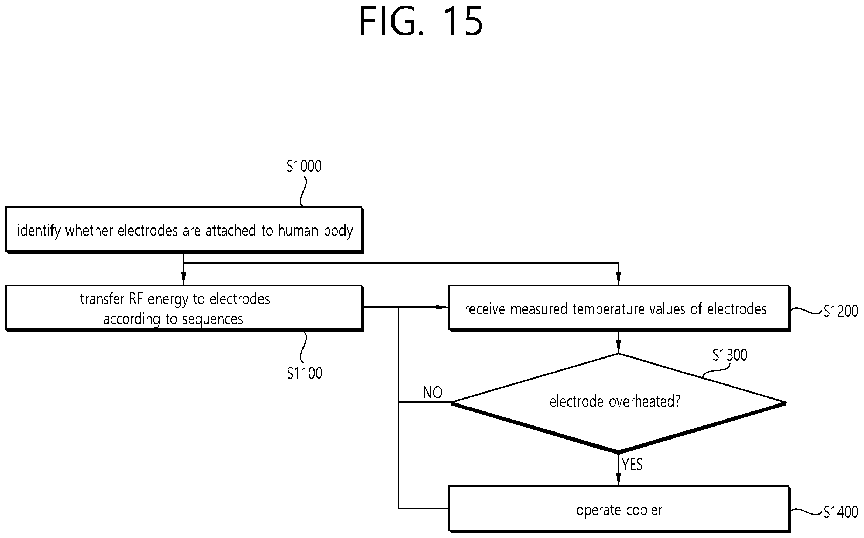

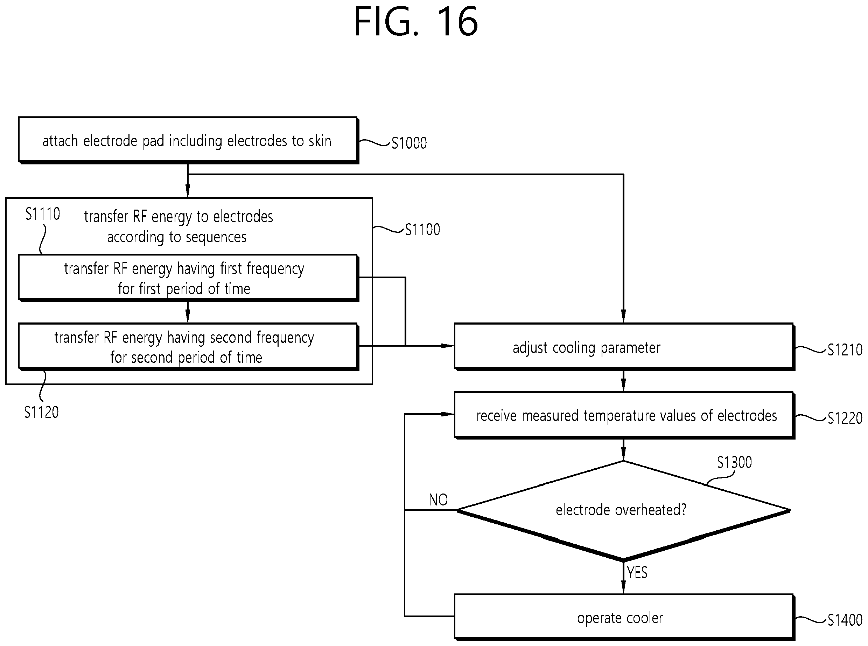

Below, a method of controlling a body contouring device using RF energy according to an embodiment of the disclosure will be described with reference to FIGS. 15 and 16.

FIG. 15 is a flowchart of a method of controlling a body contouring device using RF energy according to a fourth embodiment of the disclosure.

Referring to FIG. 15, the method of controlling the body contouring device using the RF energy according to the fourth embodiment of the disclosure includes the steps of identifying whether a plurality of electrodes are attached to a human body (S1000), transferring the RF energy to the plurality of electrodes according to sequences (S1100), receiving a measured temperature value (S1200), identifying whether the electrode is overheated (S1300), and operating the cooler (S1400).

The step S1000 of identifying whether the plurality of electrodes are attached to a human body refers to an operation of identifying whether the plurality of electrodes provided in the applicator are disposed at positions suitable for treatment. In this step S1000, impedance measurement, temperature measurement, etc. are used in identifying whether the plurality of electrodes are attached to the skin of a human body.

The step S1100 of transferring the RF energy to the plurality of electrodes according to the sequence refers to an operation of transferring the RF energy, of which the frequency and power are adjusted based on programmed treatment sequences, to the plurality of electrodes. In this case, the treatment sequences may include a sequence for pre-heating the tissue over a wide-ranging area, and a sequence for heating a deep portion of the tissue up to a treatment temperature. Each treatment sequence may be programmed in advance based on information about the age, weight, subcutaneous fat thickness, etc. of a person to be treated. The sequence may be set or updated based on a value, e.g., an impedance value measured in the step S1000 of identifying whether the plurality of electrodes are attached to a human body.

The step S1200 of receiving the measured temperature value of the electrode is performed on the premise that the electrodes are heated while the RF energy is transferred to a human body through the electrodes in the step of transferring the RF energy. In other words, the step of receiving the measured temperature value of the electrode includes receiving the measured temperature values of the plurality of electrodes. In this case, the measured temperature values may include a value obtained by measuring the temperature of the electrode where the edge effect occurs, and a value obtained by measuring the temperature of the electrode where the edge effect does not occur, among the plurality of electrodes. Meanwhile, the array of the electrodes for transferring the RF energy in this embodiment may employ the electrode pad described above with reference to FIGS. 1 to 9, and the plurality of temperature sensors may employ the temperature sensors disposed at a plurality of points on the top surface (i.e., the opposite surface to the surface where the electrodes are disposed) of the base of the electrode pad.

The step S1300 of identifying whether the electrode is overheated includes identifying whether the electrode is overheated based on the received measured temperature value. The electrode is conductive and made of metal, thereby having high heat conductivity. In other words, it will be assumed that the measured temperature is equal to the surface temperature of the skin. However, the plurality of temperature sensors calculates the temperature of the electrode based on the measured temperature of the base, and it is therefore possible to calculate the temperature of the electrode in consideration of the heat conductivity, thickness, etc. of the base. In this step, when it is identified that the temperature of at least one electrode exceeds a threshold value due to the edge effect, the cooler may operate to prevent the tissue from being damaged.

The step S1400 of operating the cooler refers to an operation of cooling the electrode by spraying the refrigerant to the electrode where the edge effect occurs. The cooler may operate to spray the refrigerant based on a preset period of time. In this case, the operation of the cooler may be performed by using a pump to spray the refrigerant, or by opening or closing a valve of a pressure tank filled with the refrigerant to spray the refrigerant.

Meanwhile, the step S1400 of operating the cooler may be performed during and/or after the step S1000 of transferring the RF energy, so as to prevent the tissue from being excessively damaged by heat of a high temperature while the body contouring device using the RF energy is operating.

FIG. 16 is a flowchart of a method of controlling a body contouring device using RF energy according to a fifth embodiment of the disclosure.

The fifth embodiment may include the same steps as those of the fourth embodiment, and repetitive descriptions about these steps will be avoided.

Referring to FIG. 16, in the method of controlling the body contouring device using the RF energy according to the fifth embodiment of the disclosure, the step S1100 of transferring the RF energy to the plurality of electrodes according to sequences may include the steps of transferring the RF energy at a first frequency for a first period of time (S1110) and transferring the RF energy at a second frequency for a second period of time (S1120).

In the sequence of transferring the RF energy, the RF energy for pre-heating the tissue is generated at the first frequency and transferred to the applicator. Here, the RF energy having the first frequency may be transferred for the first period of time. Then, the RF energy is generated at the second frequency and transferred to the applicator for the second period of time. For example, the second frequency for heating the deep portion of the tissue may be set to be higher than the first frequency.

In this case, the temperature distribution in the electrodes is varied depending on the frequencies of the RF energy, and thus the cooling parameters are adjusted to perform adequate cooling.

A step S1210 of adjusting the cooling parameters refers to an operation of adjusting the cooling parameters based on the frequency and duration time of the RF energy transferred in the step S1100 of transferring the RF energy. In this case, the cooling parameter may include parameters for adjusting the cooling quantities such as the spraying duration time, spraying pressure, etc. of the refrigerant.

For instance, the step 1210 of adjusting the cooling parameters may include increasing the spraying duration time of the refrigerant, or increasing the spraying pressure of the refrigerant so that the cooling quantity can be increased when the RF energy is transferred at the second frequency for generating deep heat.

After the cooling parameter is adjusted, the measured temperature values of the plurality of electrodes are received (S1200), it is identified whether the electrode is overheated (S1300), and the cooler is driven based on the adjusted parameters (S1400).

Although not shown, it is enough to perform the step S1210 of adjusting the cooling parameter just before driving the cooler. In other words, the step S1210 of adjusting the cooling parameter, the step S1200 of receiving the measured temperature values of the plurality of electrodes, and the step S1300 of identifying whether the electrode is overheated may be performed regardless of the sequence.

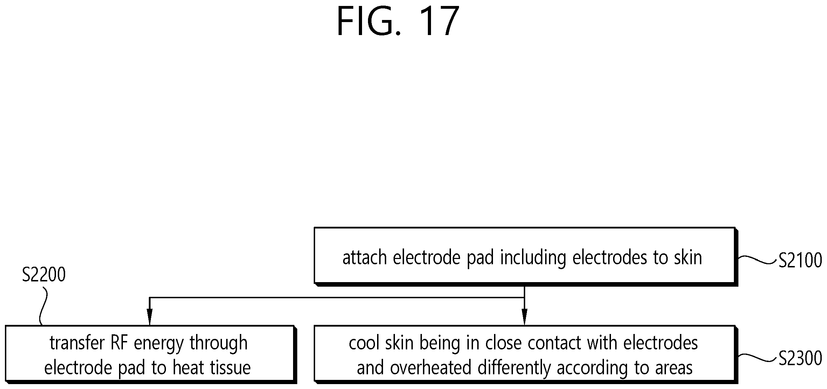

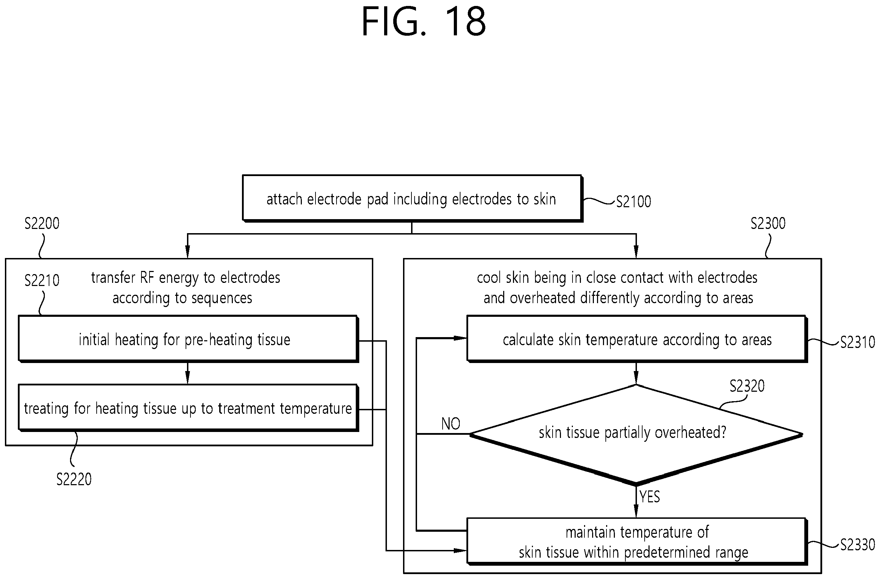

Below, a body contouring method using RF energy according to an embodiment of the disclosure will be described with reference to FIGS. 17 and 18.

FIG. 17 is a flowchart of a body contouring method using RF energy according to a sixth embodiment of the disclosure.

Referring to FIG. 17, the body contouring method using the RF energy according to the sixth embodiment of the disclosure may include the steps of attaching an electrode pad including a plurality of electrodes to skin (S2100), heating tissue by transferring the RF energy through the electrode pad (S2200), and cooling skin, overheated differently according to areas, with which the plurality of electrodes is in close contact (S2300).

The step S2100 of attaching the electrode pad including the plurality of electrodes to skin refers to an operation of attaching the applicator, on which the plurality of electrodes are arrayed, to skin, i.e., the surface of the tissue to be subjected to body contouring. The plurality of applicators may be attached to different positions so that treatment can be performed over a large area at once.

The step S2200 of heating the tissue by transferring the RF energy through the electrode pad refers to an operation of generating deep heat by transferring the generated RF energy to the tissue. In this step, the tissue may be heated up to a treatment temperature, for example, a temperature at which fat cells die.

The step S2300 of cooling the skin, overheated differently according to the areas, with which the plurality of electrodes is in close contact refers to an operation of cooling a local area on the surface of the skin, in which temperature rapidly rises due to the edge effect while each applicator transfers the RF energy the tissue. In this step, it is possible to prevent the tissue of the skin, with which the electrodes are in contact, from being damaged as temperature rapidly rises at the outer edges of the treatment area to which the RF energy is transferred. This step may be performed in such a manner that the area where the edge effect occurs is intensively cooled. For example, this step may be performed by spraying the refrigerant toward the electrode, and allowing the cooled electrode to ultimately cool the tissue of the skin. In this case, when the skin temperature exceeds a threshold value, the tissue of the skin is cooled by spraying the refrigerant for a predetermined period of time.

FIG. 18 is a detailed flowchart of the body contouring method using the RF energy according to the sixth embodiment of the disclosure.

Referring to FIG. 18, the step S2200 of transferring the RF energy to the plurality of electrodes according to the sequences may include an initial heating step S2210 of pre-heating the tissue, and a treatment step S2220 of heating the tissue at the treatment temperature.

The initial heating step S2210 of the pre-heating refers to an operation of gradually heating the tissue to be treated so that pain caused by a rapid increase in temperature can be prevented in the tissue and the tissue can be prevented from being damaged. The initial heating step S2210 of the pre-heating may be performed using the RF energy at a frequency, of which penetration into the tissue is deep and the amount of generated heat is low. For instance, this step S2210 may employ the RF energy having a frequency of 2MHz. In the initial heating step S2210 of the pre-heating, the temperature of the tissue slowly rises because difference between an amount of heat generated in the tissue and an amount of heat released by blood or the like in the tissue is not relatively large.

The treatment step S2220 of heating the tissue at the treatment temperature refers to an operation of adjusting the frequency of the RF energy to be transferred so that the temperature of the tissue rises further. In this step S2220, the frequency of the RF energy may be selected as 7 MHz or 13 MHz. As described above, a dermis layer or a fat layer may be mainly heated according to the frequencies of the RF energy selected in this step S2220. While this step is performed, the power of the RF energy may be adjusted by monitoring the impedance of the tissue. Further, the duration time of applying the RF energy may be adjusted.

The step S2300 of cooling the skin, overheated differently according to the areas, with which the plurality of electrodes is in close contact may include the steps of calculating skin temperature according to areas (S2310), identifying whether the tissue of the skin is partially overheated (S2320), and maintaining the temperature of skin tissue within a predetermined range of temperature (S2330).

The step S2310 of calculating skin temperature according to areas refers to an operation of calculating the temperature of skin, with which one applicator is in contact, according to the areas. In this step S2310, the temperature of the electrode of the applicator being in contact with the skin may be measured, or the temperature of the skin may be ultimately estimated by calculating the temperature of the electrode. In this case, the temperature is measured at a plurality of points corresponding to the areas where the plurality of electrodes are arrayed, and the temperature of the tissue is calculated with regard to the plurality of points in the tissue to which the RF energy is transferred.

The step S2320 of identifying whether the tissue of the skin is partially overheated refers to an operation of identifying whether the edge effect partially occurs as the RF energy is transferred to the tissue through the electrodes of the applicator. If the RF energy is continuously transferred even though the edge effect occurs during the transfer of the RF energy, the skin tissue may be excessively damaged. Therefore, it may be identified whether the temperature of the skin tissue corresponding to the occurrence of the edge effect exceeds the threshold value. Here, the threshold value may be set as a temperature at which the skin tissue is not denaturalized.

When it is identified that the skin tissue is partially overheated, the step S2330 of maintaining the temperature of skin tissue within a predetermined range of temperature may be performed.

This step S2330 may be performed by adjusting the cooling parameter for maintaining the temperature of skin tissue within a predetermined range of temperature according to the characteristics of the RF energy transferred in the initial heating step S2210 and the treatment step S2220. As the cooling parameter is adjusted, an area to be cooled or a cooling period of time is adjusted, thereby maintaining the temperature of the tissue within a predetermined range.

With the foregoing descriptions, a body contouring device using RF energy according to the disclosure, a control method thereof, and a body contouring method using the same make it possible to minimize unnecessary damage of tissue because electrodes are divided to have a specific shape and an area where the edge effect occurs is intensively cooled. Further, there are effect on reducing a patient's pain and maximizing treatment effect.

## Claims

1. A body contouring device comprising:
a main body configured to generate radio frequency (RF) energy; and
an applicator comprising a plurality of electrodes at one side thereof, and configured to receive the RF energy from the main body,
the applicator comprising a cooling block configured to spray a refrigerant toward outer electrodes where an edge effect occurs among the plurality of electrodes.

2. The body contouring device of claim 1, wherein the cooling block comprises:
an inlet provided at one side and introducing the refrigerant therein;
an outlet formed corresponding to an array of the outer electrodes and configured to spray the refrigerant toward the electrodes; and
an internal channel provided for fluid communication between the inlet and the outlet.

3. The body contouring device of claim 2, wherein
the outlet of the cooling block comprises a plurality of outlets, and
the internal channel is configured to have a substantially same flowing distance of the refrigerant between the inlet and each of the outlets.

4. The body contouring device of claim 3, wherein
the applicator comprises a base shaped like a flat plate,
the plurality of electrodes is provided on a bottom surface of the base,
the cooling block is provided above the base, and
the outlets of the cooling block are formed facing toward a top surface of the base.

5. The body contouring device of claim 4, wherein
the plurality of electrodes are respectively arrayed on areas divided by a plurality of virtual second lines formed along a contour path on the bottom surface of the base, and
the plurality of outlets of the cooling block are configured to spray the refrigerant toward outermost electrodes among the plurality of electrodes.

6. The body contouring device of claim 5, wherein the plurality of electrodes are respectively arranged on areas divided by a plurality of virtual first lines formed along a path intersecting the second lines from a center on the bottom surface of the base.

7. The body contouring device of claim 2, further comprising at least one temperature sensor provided on the top surface of the base.

8. The body contouring device of claim 7, wherein the temperature sensor comprises:
a first temperature sensor provided in an area which is not intensively cooled by the refrigerant; and
a second temperature sensor provided in an area which is intensively cooled by the refrigerant.

9. A method of controlling a body contouring device using radio frequency (RF) energy, comprising:
identifying whether a plurality of electrodes provided in an applicator is attached to a human body;
transferring the RF energy to the plurality of electrodes according to preset sequences;
receiving measured temperature values from at least one temperature sensor provided in the applicator and identifying whether the electrode is overheated due to an edge effect; and
operating a cooler when it is identified that the electrode is overheated,
the cooler being configured to spray a refrigerant toward the electrode where the edge effect occurs.

10. The method of claim 9, wherein the transferring the RF energy to the plurality of electrodes is performed by adjusting parameters about a frequency and/or applying period of time of the RF energy according to the preset sequences.

11. The method of claim 10, wherein the sequences are set based on the frequency of the RF energy divided into a pre-heating frequency and a high-heating frequency.

12. The method of claim 11, wherein the identifying whether the electrode is overheated is performed based on temperature values respectively measured from an area where the edge effect occurs and an area where the edge effect does not occur.

13. The method of claim 9, wherein the operating the cooler is performed while and/or after the transferring the RF energy.

14. The method of claim 13, wherein the operating the cooler is performed for an operating period of time set based on the measured temperature values.

15. The method of claim 14, wherein the operating the cooler is performed for a cooling period of time adjusted based on a frequency of the RF energy transferred in the transferring the RF energy.
